# FASCICULE DE BREVET EUROPEEN

(11) **EP 2 572 677 B1**
(45) Date de publication et mention de la délivrance du brevet: **08.07.2015**
(21) Numéro de dépôt: 12184128.2
(22) Date de dépôt: 12.09.2012
(51) Int. Cl.: A61F 2/38

(54) **Prothèse de genou de type charnière**
Scharnierknieprothese
Hinge type knee prosthesis

(30) Priorité: 21.09.2011 FR 1158415
(43) Date de publication de la demande: 27.03.2013
(73) Titulaire: SCOR GROUP, 69003 Lyon (FR)
(72) Inventeur: Bejui-Hugues, Jacques, 69006 Lyon (FR); Carret, Jean-Paul, 69006 Lyon (FR); Chatain, Frédéric, 38190 Bernin (FR); Chavane, Hervé, 69300 Caluire-et-Cuire (FR); Delalande, Jean-Luc, 45560 Saint-Denis-en-Val (FR); Denjean, Stéphane, 71960 La Roche Vineuse (FR); Gaillard, Thierry, 69003 Lyon (FR); Guyen, Olivier, 69003 Lyon (FR); Pibarot, Vincent, 38200 Vienne (FR); Tayot, Olivier, 69300 Caluire (FR)
(74) Mandataire: Le Goff, Robin

(56) Documents cités:
- EP-A1- 1 872 747
- EP-A2- 0 791 343
- DE-A1- 2 802 568
- US-A- 5 800 552

## Description

L'invention concerne une prothèse de genou de type charnière.

Dans la présente demande, on se référera au système de référence anatomique classique dans lequel le patient est debout face à un observateur. Dans ce système de référence, il est rappelé que :
- un axe vertical est un axe qui s'étend des pieds à la tête et inversement,
- un axe horizontal est un axe qui s'étend de gauche à droite et inversement,
- un axe sagittal est un axe qui s'étend du ventre vers le dos et inversement.

### ARRIERE-PLAN DE L'INVENTION

Il existe aujourd'hui trois grands groupes de prothèses de genou : la prothèse partielle, la prothèse totale et la prothèse de type charnière.

Le document US 5 800 552 décrit une prothèse de genou totale qui comprend une partie tibiale, une partie fémorale et un pivot reliant la partie tibiale à la partie fémorale. La partie tibiale comprend un orifice oblong à l'intérieur duquel le pivot, bloqué en rotation sur la partie fémorale, peut se déplacer. La partie fémorale peut donc se déplacer en rotation et en translation relativement à la partie tibiale.

La prothèse de type charnière étant bien plus contraignante que les autres groupes de prothèses, elle n'est utilisée qu'en cas de pathologies graves comme une gonarthrose complexe ou lorsque les ligaments, notamment les ligaments latéraux, ne soutiennent plus le genou.

La première génération de prothèses de type charnière n'autorisait que l'articulation du fémur sur le tibia pour permettre au patient d'étendre et de plier sa jambe. Le tibia et le fémur étaient alors étroitement liés l'un à l'autre sans aucune possibilité de mouvement latéral.

La seconde génération de prothèses de type charnière autorise, en plus de l'articulation précitée, un léger mouvement latéral du tibia relativement au fémur selon un axe vertical. La prothèse de type charnière nécessitant généralement une désinsertion des ligaments entourant le tibia et le fémur au niveau du genou, il n'y a alors pas ou peu de contrôle dudit mouvement latéral ce qui rend l'articulation formée par la prothèse peu stable. Ainsi, le mouvement latéral doit être très restreint pour limiter cette instabilité. Toutefois, même restreint, le léger mouvement latéral autorisé assure un bien plus grand confort au patient.

Une prothèse de type charnière de deuxième génération comporte habituellement une partie tibiale qui comprend une portion d'ancrage destinée à être insérée dans un tibia et un plateau tibial agencé en extrémité de la portion d'ancrage. La prothèse comporte en outre une partie fémorale qui comprend une portion d'ancrage destinée à être insérée dans un fémur associé au tibial et une tête agencée en extrémité de la portion d'ancrage, ladite tête étant conformée en une tête de fémur et comporte ainsi deux condyles. Un organe de liaison solidarise la partie tibiale à la partie fémorale par une liaison de type à cardan, l'organe de liaison comportant deux pivots. La partie fémorale est montée sur un premier pivot s'étendant selon un axe horizontal afin d'être articulée sur la partie tibiale. Le deuxième pivot, qui s'étend selon un axe vertical, est inséré dans un orifice du plateau tibial afin d'autoriser un mouvement latéral du tibia relativement au fémur.

Usuellement, le deuxième pivot est monté tournant dans un palier.

Dans tous les cas, une prothèse de type charnière nécessite généralement une désinsertion des ligaments entourant le tibia et le fémur au niveau l'articulation formée entre le tibia et le fémur de sorte que plus aucun ligament ne puisse amortir des contraintes subies par ladite articulation. Ainsi, une prothèse de type charnière s'use bien plus rapidement que les prothèses des autres groupes pour lesquels les ligaments sont encore présents après opération. En particulier, le palier a une durée de vie particulièrement faible.

### OBJET DE L'INVENTION

Un but de l'invention est de proposer une prothèse de genou de type charnière dont la durée de vie d'un palier est augmentée vis-à-vis d'une prothèse de genou de type charnière de l'art antérieur.

### BREVE DESCRIPTION DE L'INVENTION

A cet effet, on propose une prothèse de genou de type charnière comme définie dans la revendicaiton 1.

Le pivot permet donc l'articulation de la partie fémorale sur la partie tibiale selon un axe d'articulation déterminé soit selon un axe d'articulation unique. La partie fémorale ne peut ainsi que pivoter relativement à la partie tibiale au contraire de la partie fémorale de la prothèse de genou illustrée dans le document US 5 800 552.

Le palier est particulièrement sollicité dans la direction d'extension de la portion d'ancrage de la partie tibiale, soit selon un axe vertical, lorsque la prothèse est en position d'extension. Dans cette direction, l'épaisseur de la section courante du palier est minimale. Toutefois, le plateau tibial participe alors à la reprise des contraintes subies par la prothèse.

Le palier est en outre particulièrement sollicité dans la direction perpendiculaire à la direction d'extension de la portion d'ancrage de la partie tibiale, soit selon un axe sagittal, lorsque la prothèse est en position fléchie. Dans cette position, le palier reprend quasiment seul les contraintes subies par la prothèse. Cependant, l'épaisseur de la section du palier est alors maximale.

Ainsi, grâce à la forme particulière du palier, les différentes contraintes subies par la prothèse lors d'un mouvement de ladite prothèse sont bien mieux reprises par le palier ce qui limite son usure. La forme particulière du palier limite une usure du palier sans que le palier ne soit de grandes dimensions vis-à-vis des paliers de l'art antérieur.

### BREVE DESCRIPTION DES DESSINS

L'invention sera mieux comprise à la lumière de la description qui suit de modes de réalisation particuliers non limitatifs de l'invention en référence aux figures ci-jointes parmi lesquelles :
- la figure 1 est une vue en perspective d'une prothèse de genou de type charnière en position fléchie selon un premier mode de réalisation;
- la figure 2 est une vue en perspective de la prothèse illustrée à la figure 1 en position étendue ;
- la figure 3A est une vue éclatée d'une partie de l'organe de liaison de la prothèse illustrée à la figure 1;
- la figure 3B est une vue de côté schématique du palier elliptique recevant le pivot ;
- la figure 4 est une vue schématique simplifiée de face d'une prothèse de genou de type charnière en position étendue selon un deuxième mode de réalisation, le palier étant représenté en pointillés ;
- la figure 5 est une vue schématique simplifiée de face d'une prothèse de genou de type charnière en position étendue selon un troisième mode de réalisation, le palier étant représenté en pointillés;
- la figure 6 est une vue schématique simplifiée de face d'une prothèse de genou de type charnière en position étendue selon un quatrième mode de réalisation, le palier étant représenté en pointillés.

### DESCRIPTION DETAILLEE DE L'INVENTION

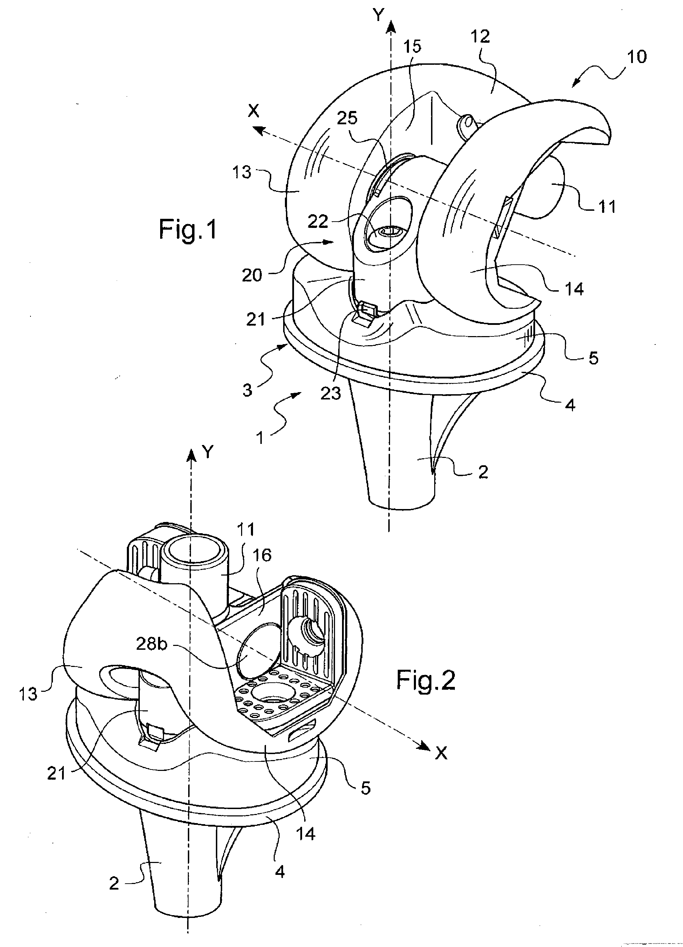

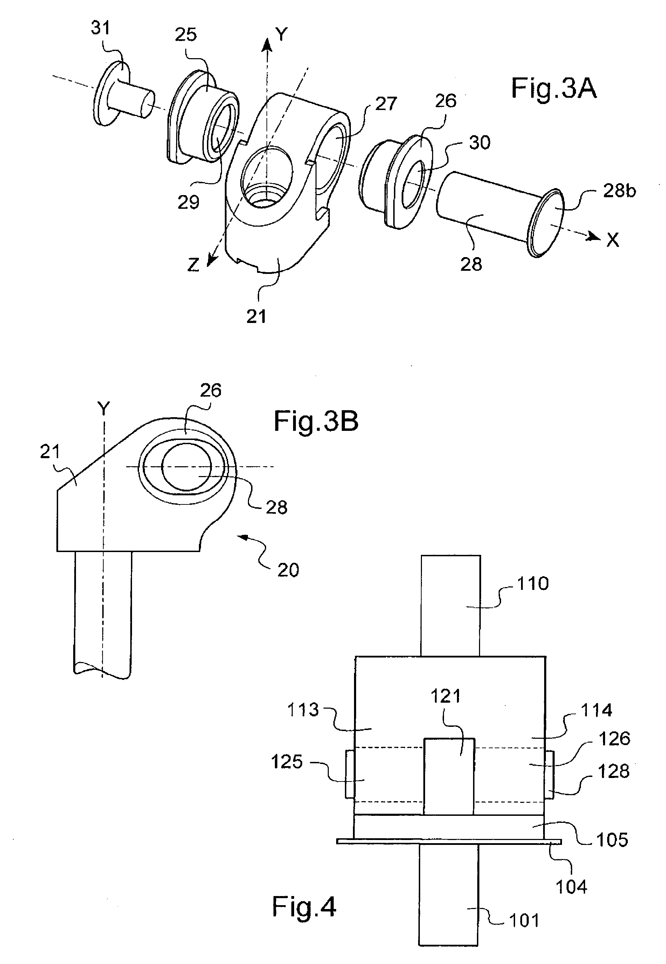

En référence aux figures 1 à 3A et 3B, la prothèse de genou de type charnière selon l'invention comporte une partie tibiale 1 qui comporte une portion d'ancrage 2 destinée à être insérée dans un tibia et un plateau tibial 3. Selon un mode de réalisation privilégié, le plateau tibial 3 comporte un sous-plateau 4 agencé en extrémité de la portion d'ancrage 2 et un sur-plateau 5 recouvrant au moins en partie le sous-plateau 4. Le sur-plateau 5 est de préférence en polyéthylène.

La prothèse de genou comporte en outre une partie fémorale 10 qui comporte une portion d'ancrage 11 destinée à être insérée dans un fémur associé au tibia et une tête fémorale 12 agencée en extrémité de ladite portion d'ancrage 11. Ici, la tête fémorale 12 est conformée en sensiblement la forme d'une tête d'un fémur et comporte ainsi un premier condyle 13 et un deuxième condyle 14. La prothèse de genou est conformée de sorte qu'au moins une portion du premier condyle 13 et une portion du deuxième condyle 14 soient en permanence en contact avec le sur-plateau 5 quelle que soit la position relative de la partie fémorale 10 vis-à-vis de la partie tibiale 1.

La prothèse de genou comporte également un premier pivot 28, dont une portion courante est ici de section circulaire, articulant la partie fémorale 10 à la partie tibiale 1, selon un axe horizontal X, perpendiculaire à la direction d'extension de la portion d'ancrage 2 de la partie tibiale.

Selon un mode de réalisation privilégié, la prothèse de genou comporte également un organe de liaison 20 pour solidariser la partie fémorale 10 à la partie tibiale 1, le premier pivot 28 étant inséré dans l'organe de liaison 20. L'organe de liaison 20 est ici conformé pour solidariser la partie fémorale 10 à la partie tibiale 1 par une liaison de type à cardan en articulant en outre la partie fémorale 10 sur la partie tibiale 1 selon un axe vertical Y, parallèle à la direction d'extension de la portion d'ancrage 2 de la partie tibiale. Un mouvement latéral de la partie tibiale 1 relativement à la partie fémorale 10 est ainsi possible. A cet effet, l'organe de liaison 20 comporte un deuxième pivot 22 qui s'étend selon l'axe vertical Y et qui est inséré dans un orifice de la partie tibiale 1. Le deuxième pivot 22 comporte ici un doigt 23 qui coopère avec une rainure de la partie tibiale 1 pour limiter le mouvement latéral de la partie tibiale 1 relativement à la partie fémorale 10 selon l'axe vertical Y.

Selon un premier mode de réalisation, l'organe de liaison 20 comporte un noyau 21 solidaire du deuxième pivot 22 de sorte que lorsque le deuxième pivot 22 est inséré dans l'orifice de la partie tibiale, le noyau 21 s'étend entre le premier condyle 13 et le deuxième condyle 14 de la partie fémorale.

Le noyau 21 de l'organe de liaison 20 comporte ici un palier dans lequel le premier pivot 28 est monté tournant qui présente un orifice cylindrique de génératrice elliptique orienté de sorte qu'un grand axe de la section elliptique s'étende selon un axe sagittal Z, perpendiculaire à la direction d'extension de la portion d'ancrage de la partie tibiale et à l'axe X, comme illustré à la figure 3B.

Le premier pivot 28 a des extrémités insérées et bloquées en rotation dans des chapes latérales ménagées dans les condyles de la partie fémorale 10.

Le palier du noyau 21 est constitué de deux douilles à épaulement 25, 26 rapportées dans l'orifice 27 d'une chape ménagée dans le noyau 21. Les deux douilles 25, 26 sont identiques et comportent chacune un épaulement venant en appui contre une face associée du noyau 21 de sorte qu'une première douille 25 soit orientée en direction du premier condyle 13 et que la deuxième douille 26 soit orientée en direction du deuxième condyle 14.

Les douilles 25, 26 présentent toutes deux un alésage interne cylindrique de génératrice elliptique, les douilles étant calées angulairement de sorte qu'un grand axe de chaque section elliptique s'étende selon l'axe sagittal Z, perpendiculaire à la direction d'extension de la portion d'ancrage de la partie tibiale.

Le premier pivot 28 s'étend ainsi selon l'axe vertical X dans un orifice 29 de la première douille 25, dans un orifice 30 de la deuxième douille 26, et dans un orifice du deuxième condyle 14 et un orifice du premier condyle 13.

De préférence, une des extrémités 28a du premier pivot 28 a une section elliptique de grand axe et de petit axe tous deux supérieurs au diamètre de la section circulaire du premier pivot 28 et au diamètre de l'autre extrémité du premier pivot 28, cette extrémité de section elliptique coopérant avec un flasque 31 de section homologue. Le flasque 31 et la première extrémité elliptique 28a du premier pivot 28 coopèrent respectivement avec une paroi externe du premier condyle 13 et une paroi externe 16 du deuxième condyle 14 pour bloquer une translation selon l'axe horizontal X du premier pivot 28.

Un deuxième mode de réalisation va être à présent décrit en référence à la figure 4. Les éléments communs avec le premier mode de réalisation illustré aux figures 1 à 3A et 3B sont une référence augmentée d'une centaine.

L'organe de liaison 120 comporte un noyau 121 et est agencé sur le plateau tibial de sorte que le noyau 121 s'étende entre le premier condyle 113 et le deuxième condyle 114 de la partie fémorale. Le premier pivot 128 s'étend alors selon l'axe vertical X dans un orifice du noyau 121 de sorte que le premier pivot 128 soit arrêté en rotation sur le noyau 121.

Le premier pivot 128 est en outre monté tournant dans un palier porté par la tête 112 de la partie fémorale 110. Le palier comporte ici une première douille 125 agencée dans un orifice du premier condyle 113 et une deuxième douille 126 agencée dans un orifice du deuxième condyle 114.

Selon l'invention, les douilles 125, 126 présentent un alésage cylindrique de génératrice elliptique, le palier étant agencé dans la partie fémorale de sorte qu'un grand axe de chaque section elliptique s'étende selon un axe sagittal, perpendiculaire à la direction d'extension de la portion d'ancrage de la partie tibiale.

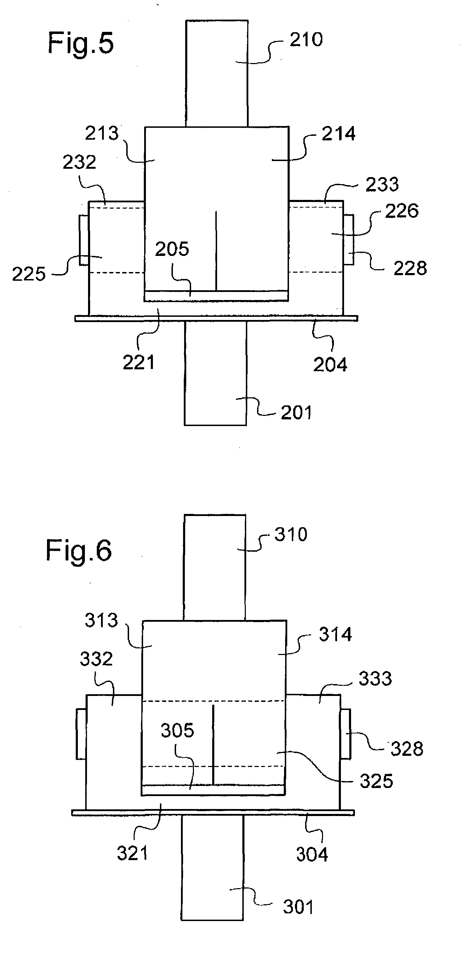

Un troisième mode de réalisation va être à présent décrit en référence à la figure 5. Les références des éléments communs avec le premier mode de réalisation illustré aux figures 1 à 3A et 3B sont augmentées de deux centaines.

L'organe de liaison 220 comporte un noyau 221 et est agencé sur le plateau tibial de sorte que le premier condyle 213 et le deuxième condyle 214 s'étendent entre un premier bras 232 et un deuxième bras 233 du noyau 221.

Le premier pivot 228 est monté tournant dans un palier porté par le noyau 221. Le palier comporte ici une première douille 225 agencée dans un orifice du premier bras 232 et une deuxième douille 226 agencée dans un orifice du deuxième bras 233.

Le premier pivot 228 s'étend alors selon l'axe vertical X dans un orifice de la première douille 225, un orifice de la deuxième douille 226 et dans un perçage du deuxième condyle 214 et un perçage du premier condyle 213 de sorte que le premier pivot 228 soit arrêté en rotation sur la partie fémorale 210.

Selon l'invention, les douilles 225, 226 présentent chacune un alésage cylindrique de génératrice elliptique, le palier étant agencé dans le noyau 221 de sorte qu'un grand axe de chaque section elliptique s'étende selon un axe sagittal, perpendiculaire à la direction d'extension de la portion d'ancrage de la partie tibiale.

Un quatrième mode de réalisation va être à présent décrit en référence à la figure 6. Les références des éléments communs avec le premier mode de réalisation illustré aux figures 1 à 3A et 3B sont augmentées de trois centaines.

L'organe de liaison 20 comporte un noyau 321 et est agencé sur le plateau tibial de sorte que le premier condyle 313 et le deuxième condyle 314 s'étendent entre un premier bras 332 et un deuxième bras 333 du noyau 321.

Le premier pivot 328 est monté tournant dans un palier porté par la tête 312 de la partie fémorale 310. Le premier pivot 328 s'étend alors selon l'axe vertical X dans un perçage du palier 325, dans un perçage du premier bras 332 et dans un perçage du deuxième bras 333 de sorte que le premier pivot 328 soit arrêté en rotation sur le noyau 321.

Selon l'invention, le palier 325 comporte une surface interne cylindrique de génératrice elliptique, le palier 325 étant agencé dans la partie fémorale de sorte qu'un grand axe de sa section elliptique s'étende selon un axe sagittal, perpendiculaire à la direction d'extension de la portion d'ancrage de la partie tibiale.

L'invention n'est pas limitée à ce qui vient d'être décrit, mais bien au contraire englobe toute variante entrant dans le cadre défini par les revendications.

En particulier, la prothèse pourra ne pas comporter d'organe de liaison distinct. La prothèse pourra ainsi comporter un pivot articulant directement la partie fémorale à la partie tibiale.

En outre, l'organe de liaison pourra être différent de celui décrit. Par exemple, l'organe de liaison pourra ne comporter que le premier pivot. L'organe de liaison pourra être porté par la partie fémorale. L'organe de liaison pourra être d'une pièce avec la partie tibiale ou la partie fémorale.

Le palier pourra être d'une pièce avec la partie fémorale, la partie tibiale ou l'organe de liaison.

Le plateau tibial pourra être d'une seule pièce qui sera, de préférence, en polyéthylène.

## Revendications

1. Prothèse de genou de type charnière comportant :
- une partie tibiale (1,101,201,301) qui comprend une portion d'ancrage (2) destinée à être insérée dans un tibia et un plateau tibial (3) agencé en extrémité de la portion d'ancrage,
- une partie fémorale (10,110,210,310) qui comprend une portion d'ancrage (11) destinée à être insérée dans le fémur,
- l'une des deux parties comportant un palier (25,26;125,126; 225, 226; 325) dans lequel un pivot (28;128;228;328) est monté;
la prothèse étant **caractérisée en ce que** le palier présente un orifice cylindrique de génératrice elliptique recevant le pivot articulant la partie fémorale à la partie tibiale selon un axe d'articulation déterminée, la génératrice elliptique étant orientée de sorte qu'un grand axe de la section elliptique soit perpendiculaire à une direction d'extension de la portion d'ancrage de la partie tibiale.

2. Prothèse de genou selon la revendication 1, dans laquelle le palier (25,26;225,226) est porté par un organe de liaison (20;220) rapporté sur le plateau tibial et le pivot (28;228) est bloqué en rotation sur la partie fémorale.

3. Prothèse de genou selon la revendication 2, dans laquelle l'organe de liaison (20) comporte un deuxième pivot (22) qui s'étend selon la direction d'extension de la portion d'ancrage de la partie tibiale (1) et qui est inséré dans la partie tibiale pour articuler en outre la partie fémorale (10) sur la partie tibiale selon un axe parallèle à ladite direction d'extension.

4. Prothèse de genou selon la revendication 2, dans laquelle l'organe de liaison comporte un noyau (21), l'organe de liaison étant agencé sur le plateau tibial de sorte que le noyau (21) s'étende entre un premier condyle (13) et un deuxième condyle (14) d'une tête de la partie fémorale (10) agencée en extrémité de la portion d'ancrage (11) de la partie fémorale, le palier (25, 26) s'étendant dans un orifice (27) du noyau (21).

5. Prothèse de genou selon la revendication 4, comportant une chape dont deux douilles (25,26) sont rapportées dans l'orifice (27) du noyau (21), l'ensemble formant le palier, les deux douilles comportant chacune un épaulement venant en appui contre une face associée du noyau (21) de sorte qu'une première douille (25) soit orientée en direction du premier condyle (13) et que la deuxième douille soit orientée en direction du deuxième condyle (14).

## Patentansprüche

1. Knieprothese vom Gelenktyp, die Folgendes beinhaltet:
- einen Tibiateil (1, 101, 201, 301), der einen Verankerungsabschnitt (2), der dazu vorgesehen ist, in eine Tibia eingesetzt zu werden, und eine Tibiaplatte (3) umfasst, die am Ende des Verankerungsabschnitts angeordnet ist,
- einen Femurteil (10, 110, 210, 310), der einen Verankerungsabschnitt (11) umfasst, der dazu vorgesehen ist, in das Femur eingesetzt zu werden,
- wobei einer der zwei Teile ein Lager (25, 26; 125, 126; 225, 226; 325) umfasst, in dem ein Drehzapfen (28; 128; 228; 328) montiert ist;
wobei die Prothese **dadurch gekennzeichnet ist, dass** das Lager eine zylindrische Öffnung mit elliptischer Mantellinie aufweist, die den Drehzapfen aufnimmt, der den Femurteil gemäß einer bestimmten Schwenkachse mit dem Tibiateil schwenkt, wobei die elliptische Mantellinie derart ausgerichtet ist, dass eine Hauptachse des elliptischen Teilabschnitts senkrecht zu einer Richtung der Erstreckung des Verankerungsabschnitts des Tibiateils ist.

2. Knieprothese nach Anspruch 1, wobei das Lager (25, 26; 225, 226) von einem Verbindungselement (20; 220) getragen wird, das auf der Tibiaplatte eingebracht ist, und der Drehzapfen (28; 228) auf dem Femurteil drehblockiert ist.

3. Knieprothese nach Anspruch 2, wobei das Verbindungselement (20) einen zweiten Drehzapfen (22) beinhaltet, der sich gemäß der Richtung der Erstreckung des Verankerungsabschnitts des Tibiateils (1) erstreckt und der in den Tibiateil eingesetzt wird, um außerdem den Femurteil (10) auf dem Tibiateil gemäß einer Achse zu schwenken, die parallel zu der besagten Erstreckungsrichtung ist.

4. Knieprothese nach Anspruch 2, wobei das Verbindungselement einen Kern (21) beinhaltet, wobei das Verbindungselement derart auf der Tibiaplatte angeordnet ist, dass der Kern (21) sich zwischen einem ersten Kondylus (13) und einem zweiten Kondylus (14) eines Kopfs des Femurteils (10) erstreckt, der am Ende des Verankerungsabschnitts (11) des Femurteils angeordnet ist, wobei das Lager (25, 26) sich in eine Öffnung (27) des Kerns (21) erstreckt.

5. Knieprothese nach Anspruch 4, die eine Abdeckung beinhaltet, deren zwei Buchsen (25, 26) in der Öffnung (27) des Kerns (21) eingebracht sind, wobei die Einheit das Lager bildet, wobei die zwei Buchsen jeweils einen Absatz beinhalten, der gegen eine Fläche aufzuliegen kommt, die mit dem Kern (21) derart in Zusammenhang steht, dass eine erste Buchse (25) in der Richtung des ersten Kondylus (13) ausgerichtet ist und dass die zweite Buchse in der Richtung des zweiten Kondylus (14) ausgerichtet ist.

## Claims

1. A hinge-type knee prosthesis including:
- a tibial part (1, 101, 201, 301) which comprises an anchoring portion (2) intended to be inserted in a tibia and a tibial tray (3) arranged at the end of the anchoring portion,
- a femoral part (10, 110, 210, 310) which comprises an anchoring portion (11) intended to be inserted in the femur,
- one of the two parts including a bearing (25, 26; 125, 126; 225, 226; 325) wherein a pivot (28; 128; 228; 328) is mounted;
the prosthesis being **characterized in that** the bearing presents a cylindrical orifice of elliptical generator receiving the pivot articulating the femoral part to the tibial part according to a determined articulation axis, the elliptical generator being oriented so that a major axis of the elliptical section is perpendicular to a direction of extension of the anchoring portion of the tibial part.

2. The knee prosthesis according to claim 1, wherein the bearing (25, 26; 225, 226) is carried by a connecting member (20; 220) arranged on the tibial tray and the pivot (28; 228) is blocked in rotation on the femoral part.

3. The knee prosthesis according to claim 2, wherein the connecting member (20) includes a second pivot (22) which extends according to the direction of extension of the anchoring portion of the tibial part (1) and which is inserted into the tibial part in order to further articulate the femoral part (10) on the tibial part according to an axis parallel to said direction of extension.

4. The knee prosthesis according to claim 2, wherein the connecting member includes a core (21), the connecting member being arranged on the tibial tray so that the core (21) extends between a first condyle (13) and a second condyle (14) of a head of the femoral part (10) arranged at the end of the anchoring portion (11) of the femoral part, the bearing (25, 26) extending in an orifice (27) of the core (21).

5. The knee prosthesis according to claim 4, including a clevis, two sockets of which (25, 26) are brought in the orifice (27) of the core (21), the assembly forming the bearing, the two sockets each including a shoulder bearing against an associated face of the core (21) so that a first socket (25) is oriented in the first condyle (13) direction and that the second socket is oriented in the second condyle (14) direction.
